# EUROPEAN PATENT SPECIFICATION

(11) **EP 1 507 511 B1**
(45) Date of publication and mention of the grant of the patent: **26.07.2006**
(21) Application number: 03737971.6
(22) Date of filing: 22.05.2003
(51) Int. Cl.: A61Q 19/00, A61K 8/02, C08F 8/44

(54) **PERSONAL CARE COMPOSITIONS WITH HYDROXY AMINE NEUTRALIZED POLYMERS**
KÖRPERPFLEGEZUSAMMENSETZUNGEN ENTHALTEND MIT HYDROXYLAMIN NEUTRALISIERTEN POLYMEREN
COMPOSITIONS DE SOINS PERSONNELS A POLYMERES NEUTRALISES PAR HYDROXY AMINES

(30) Priority: 29.05.2002 US 383850 P
(43) Date of publication of application: 23.02.2005
(73) Proprietor: UNILEVER PLC, London EC4P 4BQ (GB); UNILEVER N.V., 3013 AL Rotterdam (NL)
(72) Inventor: FARYNIARZ, Joseph Raymond, 40 Merritt Boulevard, Trumbull, CT 06611 (US); ZHANG, Joanna Hong, 40 Merritt Boulevard, Trumbull, CT 06611 (US); MINER, Philip Edward, 40 Merritt Boulevard, Trumbull, CT 06611 (US)
(74) Representative: Acham, Nicholas Clive
(86) International application number: PCT/EP2003/005466
(87) International publication number: WO 2003/099253

(56) References cited:
- US-A- 3 738 991
- US-A- 3 766 116
- US-A- 6 063 398
- US-A1- 2002 041 858
- US-A1- 2002 176 834
- US-B1- 6 344 218
- SCHRADER K.: 'Grundlagen und Rezepturen der Kosmetika', 1989, HÜTHIG BUCH VERLAG HEIDELBERG * page 750 - page 751 *
- "Luviset CA66 Technical Data Sheet" 1999, BASF FINE CHEMICALS * page 1 - page 2 *
- "International Cosmetic Ingredient Dictionary" 1995, THE COSMETIC, TOILETRY AND FRAGRANCE ASSOCIATION 6 page 42

## Description

The invention concerns personal care compositions containing acid functionalized polymers neutralized by hydroxy amines.

Moisturizing agents are present in many personal care products. These agents appear in a variety of chemical structures. Many polyols are operative for this purpose. Glycerin is particularly prominent. Polymeric polyols have also been utilized such as polyethylene glycols and sorbitol. Alphahydroxy acids such as lactic acid as well as polymeric forms including polylactic acid have been identified as functioning to moisture skin. While all the aforementioned materials are effective, there is a continuing search for new compounds which may have moisturizing properties.

A hair styling composition comprising cross linked polyacrylic acid and amino methyl propanol (AMP) are disclosed in US 2002/0041858 A.

Hair-setting and blow-waiving compositions comprising AMP and a copolymer of vinyl acetate and crotonic acid are disclosed on p. 750 of "Grundlagen und Rezepturen der Kosmetika" (K. Schrader, 2^{nd} edition, Heidelberg: Hüthig, 1989).

Skin deodorizing and sanitizing compositions comprising crosslinked polyacrylic acid and AMP are disclosed in US 6,344,218.

In a first aspect, there is provided a personal care composition which includes:
(i) from about 0.000001% to about 10% by weight of a polymer having a plurality of acid groups at least partially neutralized by a mono-hydroxy substituted amine, the amine in non-protonated form having general formula I given herein below; and
(ii) from about 1% to about 99.9% by weight of a cosmetically acceptable carrier.

Furthermore, the present invention provides a set of new polymers. In particular, the dimethylaminoethanol salts of acid functionalized polymers have proven very effective.

We have now found a class of polymers functionalized with salt groups which are effective at moisturization. The salts of these polymers arise from neutralization of acid groups on the precursor polymer by a mono-hydroxy substituted amine. The amine in non-protonated form has a general formula I: 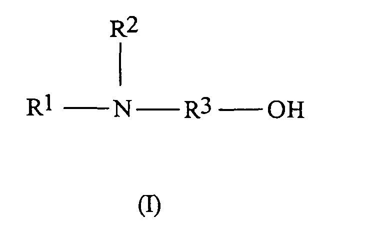
wherein R¹ and R² are branched or unbranched C₁-C₃₀ radicals selected from the group consisting of alkyl, cycloalkyl, alkenyl, aryl, alkylaryl, alkoxyalkyl and combinations thereof;
R³ is a branched or unbranched C₁-C₃₀ radical selected from the group consisting of alkylene, cycloalkylene, arylene and combinations thereof; and
R¹ with R² can optionally form a ring and independently R¹ with R³ can form a ring.

Illustrative mono-hydroxy amines include dimethylaminoethanol, diethylaminoethanol, diisopropylaminoethanol, ethylmethylamino- ethanolamine, methylbutylaminoethanolamine, dimethylamino- methanol, diethylaminomethanol, methylethylaminomethanol, dimethylaminopropanol, diethylaminopropanol, dipropylaminopropanol, ethylpropylaminopropanol, diphenylaminoethanol, methyl- phenylaminoethanol, para-toluoylmethylaminoethanol, dimethylaminophenylethanol, piperidinylethanol, 2-methylpyridinyl- ethanol, 2-pyrrolidonylethanol and pyrrolylethanol. Most preferred is dimethylaminoethanol (DMAE).

Amounts of the amine neutralized polymer may range from about 0.000001% to about 10%, preferably from about 0.00001% to about 1%, more preferably from about 0.0001% to about 0.5%, optimally from about 0.01% to about 0.1% by weight of the composition.

Polymers of the present invention may have a molecular weight ranging from 1,000 to 60 million, preferably from 5,000 to 30 million, optimally from 30,000 to 2 million average number molecular weight. Polymers of this invention may be homopolymers or copolymers with at least two different monomers forming the copolymer. Included among the polymers are those which are crosslinked and those which are non-crosslinked. At least one of the monomer units or crosslinking monomer units must contain an acid moiety which can be neutralized with the mono-hydroxy amine. Acid moieties may include carboxylic acid, phosphonic acid, phosphoric acid, sulphonic acid, sulfuric acid and sulfinic acid. The polymer will contain at least one monomer unit with a neutralizable acid function.

Acid functionalized monomers may comprise monoethylenically unsaturated C₃-C₅ carboxylic acids. Particularly suitable for the present invention are acrylic acid, methacrylic acid, crotonic acid, itaconic acid, maleic acid, fumaric acid and combinations thereof. Monomers with acid units may also include 2-acrylamido-2-methylpropane sulphonic acid and 2-methyl-2-[(1-oxo-2-propenyl)amino]-1-propane sulphonic acid.

Monomers other than those requiring acid neutralization may be copolymerized with the acid funcationalized ones. These monomers include: methylmethacrylate, ethylacrylate, ethylmethacrylate, N-propylacrylate, N-propylmethacrylate, t-butyl acrylate, t-butyl methacrylate, isobutyl acrylate, isobutyl methacrylate and combinations thereof.

Heterocyclic nitrogen monomers which may be suitable for copolymerization include N-vinyl-substituted lactams such as N-vinyl pyrrolidone, N-vinylvalerolactam and N-vinylcaprolactam and combinations thereof.

Amides may also be suitable copolymerizable monomers for the present invention. Representative amides include acrylamides which are unsubstituted or N-substituted by C₁-C₈ -alkyl or - hydroxyalkyl groups. Illustrative are acrylamide, methacrylamide, N-methylacrylamide, N-methylmethacrylamide, N-ethylacrylamide, N-ethylmethacrylamide, N-hydroxypropylacrylamide, N-hydroxyethylmethacrylamide and combinations thereof.

Monomers of the C₁-C₃₀ vinyl ester type may also be copolymerized. Illustrative are vinyl acetate, vinyl propionate, vinyl butyrate, vinyl 2-ethylhexanoate, vinyl palmitate, vinyl stearate, vinyl formamide, methyl diglycol vinyl ether and combinations thereof.

Monomers based on styrene may also be employed. Illustrative are styrene, p-halostyrene, p-methylstyrene, alpha-methylstyrene and combinations thereof.

When polymers of the present invention are crosslinked, the crosslink agents are monomers having at least two olefinic double bonds. Illustrative are allyl sucrose ether, ethylene glycol methacrylate, diallyloxyacetate, diethylene glycol diacrylate, diallylurea, trimethylopropane triacrylate, dipropylene glycol diallyl ether, polyglycol diallyl ether, triethylene glycol divinyl ether, hydroquinone diallyl ether, tetraethylene glycol diacrylate, triallyl amine, trimethylolpropane diallyl ether, methylenebisacrylamide, divinylbenzene and combinations thereof.

Particularly preferred polymers include Carbopol® 940 and Carbopol® Ultrez Z 10 which are polyacrylates crosslinked with diallyl monomer; polyacrylic acid; poly(acrylic acid/acryloyldimethyl) taurate; hydroxyethylacrylate/acryloyldimethyl taurate; poly(acrylic acid/acrylamide); acryloyldimethyl taurate/vinyl pyrrolidone; poly(acryloyldimethyl taurate/vinylformamide); and polyacrylamide crosslinked with acryloyldimethyl taurate.

Most preferred are Carbomers. This category are polymers formed from free radical polymerization of acrylic acid crosslinked with allyl ethers of sucrose of pentaerytritol. They are available as Carbopol® 900 series which includes 910, 934, 940, 941, 954, 980 and 981, all sold by the B.F. Goodrich Company. Copolymers known as acrylates/C₁₀₋₃₀ alkyl acrylate crosspolymers commercially available as Carbopol® 1382 or Pemulen TR-1 may also be useful, but are less preferred because of the hydrophobic C₁₀₋₃₀ alkyl acrylate chain.

For purposes of the present invention, not all acid groups of the polymer need be neutralized by the hydroxy amine. Some of the acid or anhydride groups may be left unneutralized or neutralized with another alkaline material. Sodium, potassium, triethanolammonium and ammonium cations may also co-saltify the polymeric acid units. Accordingly, the invention contemplates a polymer wherein from about 0.1% to about 100% of the acid units are neutralized with a mono-hydroxy amine, preferably from about 10% to about 90%, optimally about 50% to about 80%.

Compositions of this invention may have a pH ranging from about 2.5 to about 9.5, preferably from about 3 to about 8, optimally from about 4 to less than about 7.

Compositions of this invention will also include a cosmetically acceptable carrier. Amounts of the carrier may range from 1% to 99.9%, preferably from about 70% to about 95%, optimally from about 80% to about 90%. Among the useful carriers are water, emollients, fatty acids, fatty alcohols, humectants, thickeners and combinations thereof.

The carrier may be aqueous, anhydrous or an emulsion. Preferably the compositions are aqueous, especially water and oil emulsions of the W/O or O/W variety. Water when present may be in amounts ranging from about 5% to about 95%, preferably from about 20% to about 70%, optimally from about 35% to about 60% by weight.

Emollient materials may serve as cosmetically acceptable carriers. These may be in the form of silicone oils, synthetic esters and hydrocarbons. Amounts of the emollients may range anywhere from about 0.1% to about 95%, preferably between about 1% and about 50% by weight.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Nonvolatile silicone oils useful as an emollient material include polyalkyl siloxanes, polyalkylaryl siloxanes and polyether siloxane copolymers. The essentially nonvolatile polyalkyl siloxanes useful herein include, for example, polydimethyl siloxanes with viscosities of from about 5 x 10⁻⁶ to 0.1 m²/s at 25°C. Among the preferred non-volatile emollients useful in the present compositions are the polydimethyl siloxanes having viscosities from about 1 x 10⁻⁵ to about 4 x 10⁻⁴ m²/s at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among suitable ester emollients are included:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
(4) Wax esters such as beeswax, spermaceti wax and tribehenin wax.
(5) Sterols esters, of which cholesterol fatty acid esters are examples thereof.
(6) Sugar ester of fatty acids such as sucrose polybehenate and sucrose polycottonseedate.

Hydrocarbons which are suitable cosmetically acceptable carriers include petrolatum, mineral oil, C₁₁-C₁₃ isoparaffins, polyalphaolefins, and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, hydroxystearic, oleic, linoleic, ricinoleic, arachidic, behenic and erucic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5% to 50%, preferably between 1% and 15% by weight of the composition.

Thickeners can be utilized as part of the cosmetically acceptable carrier of compositions according to the present invention. Typical thickeners include cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums.

Inorganics may also be utilized as thickeners, particularly clays such as bentonites and hectorites, fumed silicas, and silicates such as magnesium aluminum silicate (Veegum® ). Amounts of the thickener may range from 0.0001% to 10%, usually from 0.001% to 1%, optimally from 0.01% to 0.5% by weight.

Cosmetic compositions of the present invention may be in any form. These forms may include lotions, creams, roll-on formulations, sticks, mousses, aerosol and non-aerosol sprays, adhesive patch and pad-applied formulations.

Surfactants may also be present in cosmetic compositions of the present invention. Total concentration of the surfactant when present may range from about 0.1% to about 40%, preferably from about 1% to about 20%, optimally from about 1% to about 5% by weight of the composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionate, C₈-C₂₀ alkyl ether phosphates, C₈₋C₂₀ sarcosinates and combinations thereof.

Sunscreen actives may also be included in compositions of the present invention. Particularly preferred are such materials as ethylhexyl p-methoxycinnamate, available as Parsol MCX® , Avobenzene, available as Parsol 1789® and benzophenone-3, also known as Oxybenzone. Inorganic sunscreen actives may be employed such as microfine titanium dioxide, zinc oxide, polyethylene and various other polymers. Amounts of the sunscreen agents when present may generally range from 0.1% to 30%, preferably from 2% to 20%, optimally from 4% to 10% by weight.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability.

Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

Compositions of the present invention may also contain vitamins. Illustrative water-soluble vitamins are Niacinamide, Vitamin B₂, Vitamin B₆, Vitamin C and Biotin. Among the useful water-insoluble vitamins are Vitamin A (retinol), Vitamin A Palmitate, Ascorbyl Tetraisopalmitate, Vitamin E (tocopherol), Vitamin E Acetate and DL-panthenol. Total amount of vitamins when present in compositions according to the present invention may range from 0.001% to 10%, preferably from 0.01% to 1%, optimally from 0.1% to 0.5% by weight.

Another adjunct ingredient can be that of an enzyme. Particularly preferred is superoxide dismutase, commercially available as Biocell SOD from the Brooks Company, USA.

Skin lightening agents may be included in the compositions of the invention. Illustrative substances are placental extract, lactic acid, niacinamide, arbutin, kojic acid, resorcinol and derivatives including 4-substituted resorcinols and combinations thereof. Amounts of these agents may range from about 0.1% to about 10%, preferably from about 0.5% to about 2% by weight of the compositions.

Desquamation agents are further optional components. Illustrative are the alpha-hydroxycarboxylic acids and beta-hydroxycarboxylic acids. Among the former are salts of glycolic acid, lactic acid and malic acid. Salicylic acid is representative of the beta-hydroxycarboxylic acids. Amounts of these materials when present may range from about 0.1% to about 15% by weight of the composition.

A variety of herbal extracts may optionally be included in compositions of this invention. Illustrative are green tea, chamomile, licorice and extract combinations thereof. The extracts may either be water soluble or water-insoluble carried in a solvent which respectively is hydrophilic or hydrophobic. Water and ethanol are the preferred extract solvents.

Anti-microbial agents may also be included in the compositions of this invention. Illustrative are trichlosan, trichlocarban, octopyrox® and zinc pyrithione. Amounts may range from about 0.01% to about 5%, preferably from about 0.1% to about 0.5% by weight of the composition.

Colorants, fragrances, opacifiers and abrasives may also be included in compositions of the present invention. Each of these substances may range from about 0.05% to about 5%, preferably between 0.1% and 3% by weight.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

### EXAMPLES

Except in the operating and comparative examples, or where otherwise explicitly indicated, all numbers in this description indicating amounts of material ought to be understood as modified by the word "about".

The following examples will more fully illustrate the embodiments of this invention. All parts, percentages and proportions referred to herein and in the appended claims are by weight unless otherwise illustrated.

### Example 1

A typical skin cream according to the present invention formulated with the dimethylaminoethanol salt of Carbopol® 940 as described in the formula under Table I.

**TABLE I**

| INGREDIENT | WEIGHT% |
|---|---|
| PHASE A | |
| Water | Balance |
| Disodium EDTA | 0.05 |
| Methyl Paraben | 0.15 |
| Magnesium Aluminum Silicate | 0.60 |
| Triethanolamine | 1.20 |

| PHASE B | |
|---|---|
| Xanthan Gum | 0.20 |
| Natrosol® 250HHR (ethyl cellulose) | 0.50 |
| Butylene Glycol | 3.00 |
| Glycerin | 2.00 |
| DMAE Salt of Carbopol® 940 (2% Active) | 10.00 |

| PHASE C | |
|---|---|
| Sodium Stearoyl Lactylate | 0.10 |
| Glycerol Monostearate | 1.50 |
| Stearyl Alcohol | 1.50 |
| Isostearyl Palmitate | 3.00 |
| Silicone Fluid | 1.00 |
| Cholesterol | 0.25 |
| Sorbitan Stearate | 1.00 |
| Butylated Hydroxy Toluene | 0.05 |
| Vitamin E Acetate | 0.01 |
| PEG-100 Stearate | 2.00 |
| Stearic Acid | 3.00 |
| Propyl Paraben | 0.10 |
| Parsol MCX® | 2.00 |
| Caprylic/Capric Triglyceride | 0.50 |
| Hydroxycaprylic Acid | 0.01 |
| C12-15 Alkyl Octanoate | 3.00 |

| PHASE D | |
|---|---|
| Vitamin A Palmitate | 0.10 |
| Bisabolol | 0.01 |
| Vitamin A Acetate | 0.01 |
| Fragrance | 0.03 |
| Retinol 50C | 0.02 |

### Example 2

A water-in-oil topical liquid make-up foundation utilizing the polymers of the present invention as described in Table II below.

**TABLE II**

| INGREDIENT | WEIGHT % |
|---|---|
| PHASE A | |
| Cyclomethicone | 9.25 |
| Cetyl Octanoate | 2.00 |
| Dimethicone Copolyol | 20.00 |

| PHASE B | |
|---|---|
| Talc | 3.38 |
| Pigment (Iron Oxides) | 10.51 |
| Spheron L-1500 (Silica) | 0.50 |

| PHASE C | |
|---|---|
| Synthetic Wax Durachem 0602 | 0.10 |
| Arachidyl Behenate | 0.30 |

| PHASE D | |
|---|---|
| Cyclomethicone | 1.00 |
| Trihydroxystearin | 0.30 |

| PHASE E | |
|---|---|
| Laureth-7 | 0.50 |
| Propyl Paraben | 0.25 |

| PHASE F | |
|---|---|
| Fragrance | 0.05 |

| PHASE G | |
|---|---|
| Water | balance |
| DMAE Malonate | 3.00 |
| DMAE Salt of Aristoflex® AVC | 1.00 |
| DMAE Salt of Carbopol® 940 (2% active) | 10.00 |
| Methyl Paraben | 0.12 |
| Propylene Glycol | 8.00 |
| Niacinamide | 4.00 |
| Glycerin | 3.00 |
| Sodium Chloride | 2.00 |
| Sodium Dehydroacetate | 0.30 |

### Example 3

Illustrated herein is a skin cream incorporating the polymeric salts of the present invention.

**TABLE III**

| INGREDIENT | WEIGHT % |
|---|---|
| Glycerin | 6.93 |
| Niacinamide | 5.00 |
| DMAE Malonate | 5.00 |
| Permethyl 101 A¹ | 3.00 |
| DC-1403² | 2.00 |
| Isopropyl Isostearate | 1.33 |
| Arlatone 2121³ | 1.00 |
| Cetyl Alcohol CO-1695 | 0.72 |
| SEFA Cottonate⁴ | 0.67 |
| Tocopherol Acetate | 0.50 |
| Panthenol | 0.50 |
| Stearyl Alcohol | 0.48 |
| Titanium Dioxide | 0.40 |
| Disodium EDTA | 0.10 |
| Glydant Plus⁵ | 0.10 |
| PEG-100 Stearate | 0.10 |
| Stearic Acid | 0.10 |
| DMAE Salt of Carbopol® Ultrez 10 | 0.10 |
| Purified Water | Balance |

| | |
|---|---|
| ¹ Isohexadecane, Presperse Inc., South Plainfield, NJ | |
| ² dimethicone(and)dimethiconol, Dow Corning Corp. Midland, MI | |
| ³ Sorbitan Monostearate and Sucrococoate, ICI Americas Inc., Wilmington, DE | |
| ⁴ Sucrose ester of fatty acid | |
| ⁵ DMDM Hydantoin (and) Iodopropynyl Butylcarbamate, Lonza Inc., Fairlawn, NJ | |

### Example 4

Illustrative of another cosmetic composition according to the present invention is the formula of Table IV.

**TABLE IV**

| INGREDIENT | WEIGHT % |
|---|---|
| Polysilicone-11 | 22.5 |
| Cyclomethicone | 59 |
| Petrolatum | 11 |
| DMAE Carbopol® 980 (2% in water) | 7 |
| Dimethicone Copolyol | 0.4 |
| Retinoxytrimethylsilane (Silcare® 1M-75) | 0.1 |

### Example 5

A relatively anhydrous composition according to the present invention is reported in Table V.

**TABLE V**

| INGREDIENT | WEIGHT % |
|---|---|
| Cyclomethicone | 80.65 |
| Dimethicone | 9.60 |
| Squalane | 6.00 |
| Isostearic Acid | 1.90 |
| Borage Seed Oil | 0.90 |
| DMAE Salt of Carbopol® Ultrez 10 | 0.50 |
| Retinyl Palmitate | 0.25 |
| Ceramide 6 | 0.10 |
| Tocopherol | 0.10 |

### Example 6

An aerosol packaged foaming cleanser suitable for the present invention is outlined in Table VI.

**TABLE VI**

| INGREDIENT | WEIGHT % |
|---|---|
| Sunflower Seed Oil | 20.00 |
| Maleated Soybean Oil | 5.00 |
| Silicone Urethane | 1.00 |
| Polyglycero-4 Oleate | 1.00 |
| Sodium C14-16 Olefin Sulfonate | 15.00 |
| Sodium Lauryl Ether Sulphate (25% active) | 15.00 |
| Cocoamidopropylbetaine | 15.00 |
| DC 1784® (Silicone Emulsion 50%) | 5.00 |
| Polyquaternium-11 | 1.00 |
| DMAE Salt of Aristoflex AVC® | 1.00 |
| Water | Balance |

An aerosol was prepared using 92% by weight of the concentrate in Table VI and 8% propellant, the latter being a combination of dimethylether, isobutane and propane.

### Example 7

An adhesive cosmetic patch may also be formulated according to the present invention. An adhesive hydrogel is prepared by mixing 30 grams of 2-acrylamido-2-methylpropane sulphonic acid monomer in 20 grams distilled water and 5 grams of a 1% aqueous solution of methylene-bis-acrylamide. The solution is then activated with 0.4% magnesium persulphate catalyst. Shortly after mixing the catalyst with the hydrogel solution, 0.1 grams DMAE in 5 ml water is added. The resultant solution is coated onto a 50/50 blend of polypropylene and hydrophilic polyester and allowed to solidify. The resulting deposited hydrogel is warmed for 24 hours at 40°C in a hot air oven. Final water content of the hydrogel is 50%. A polystyrene backing layer is laid over the adhesive hydrogel.

### Example 8

A disposable, single use personal towelette product is described according to the present invention. A 70/30 polyester/rayon non-woven towelette is prepared with a weight of 1.8 grams and dimensions of 15 cm by 20 cm. Onto this towelette is impregnated a composition as outlined in Table VII below.

**TABLE VII**

| INGREDIENT | WEIGHT % |
|---|---|
| DMAE Salt of Carbopol® 940 (2% Active in water) | 7.50 |
| Glycerin | 2.00 |
| Hexylene Glycol | 2.00 |
| Disodium Capryl Amphodiacetate | 1.00 |
| Gluconolactone | 0.90 |
| Silicone Microemulsion | 0.85 |
| Witch Hazel | 0.50 |
| PEG-40 Hydrogenated Castor Oil | 0.50 |
| Fragrance | 0.20 |
| Vitamin E Acetate | 0.001 |
| Water | Balance |

### Example 9

A set of comparative experiments were conducted to evaluate the relative Moisture Vapor Transmission (MVT) of polymeric salts according to the present invention. Samples of a neutralized polyacrylic polymer were compared to the same polymer neutralized with DMAE and a polymer sample neutralized with ammonia. The procedure involved twenty-four pieces of untreated porcine skin. These were acclimatized for twenty-four hours to determine the "before" MVT of each piece. Thereafter, 0.1 ml of each sample solution was applied uniformly to eight pieces of skin and dried overnight at 33°C. The "after" MVT of each piece was measured during the next twenty-four hours. The percent change in MVT of each piece resulting from the treatment was calculated. Table VIII details the results.

**TABLE VIII Moisture Vapor Transmission Test**

| SAMPLE NO. | POLYMER** | % REDUCTION IN MVT* |
|---|---|---|
| 1 | Polyacrylic acid | 0.05 +/- 1.98 |
| 2 | DMAE Polyacrylate | 7.48 +/- 1.76 |
| 3 | Ammonium Polyacrylate | 3.21 +/- 1.58 |

| | | |
|---|---|---|
| * Mean +/- 95% confidence interval | | |
| ** Neutralized and unneutralized polyacrylic acid utilzed as 50% aqueous solution. | | |

An occlusive film will reduce the MVT, resulting in reduced moisture loss through the skin. Products that are occlusive make good moisturizers. The results show that at the 95% confidence interval, the DMAE neutralized polyacrylic acid created an occlusive film that was significantly different from the non-neutralized polymer (Sample No. 1) and the ammonia neutralized polymer (Sample No. 3). Conversely, neither the non-neutralized nor the ammonia neutralized polymer films signficantly altered the Moisture Vapor Transmission. The conclusion is that the DMAE neutralized polyacrylic acid (Sample No. 2) forms an occlusive film that reduces the in vitro moisture loss through skin.

The foregoing description and examples illustrate selected embodiments of the present invention. In light thereof variations and modifications will be suggested to one skilled in the art, all of which are within the spirit and purview of this invention.

## Claims

1. A cosmetic composition comprising:
(i) from about 0.000001% to about 10% by weight of a polymer having a plurality of acid groups at least partially neutralized by a mono-hydroxy substituted amine, the amine in non-protonated form having general formula I: 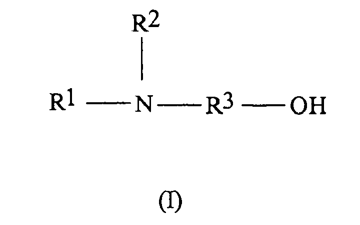
wherein R¹ and R² are branched or unbranched C₁-C₃₀ radicals selected from the group consisting of alkyl, cycloalkyl, alkenyl, aryl, alkylaryl, alkoxyalkyl and combinations thereof;
R³ is a branched or unbranched C₁-C₃₀ radical selected from the group consisting of alkylene, cycloalkylene, arylene and combinations thereof; and
R¹ with R² can optionally form a ring and independently R¹ with R³ can form a ring; and
(ii) from 1 to 99.9% by weight of a cosmetically acceptable carrier.

2. The composition according to claim 1 wherein the amine is dimethylaminoethanol.

3. The composition according to claim 1 or claim 2 wherein the pH ranges from about 4 to less than about 7.

4. The composition according to any of the preceding claims wherein the polymer is formed from a vinyl monomer selected from acrylic acid, methacrylic acid, crotonic acid, itaconic acid, maleic acid, fumaric acid, 2-acrylamido-2-methylpropane-sulfonic acid, 2-methyl-2-[(1-oxo-2-propenyl)amino]-1-propane sulphonic acid and mixtures thereof.

5. The composition according to claim 4 wherein the polymer further is formed from a co-monomer selected from methylmethacrylate, ethyl acrylate, t-butyl acrylate, t-butylmethacrylate, N-vinylpyrrolidone, acrylamide, hydroxyethylacrylate, N-hydroxypropylacrylamide, vinyl acetate, vinyl formamide, styrene, allyl sucrose ether, divinyl benzene, methylenebisacrylamide and mixtures thereof.

6. An adhesive cosmetic patch or a disposable towelette product containing a cosmetic composition according to any of the preceding claims.

7. The composition according to any one of claims 1 to 5 wherein the carrier is a silicone oil.

8. The composition according to any one of claims 1 to 5 further comprising a substance selected from the group consisting of sunscreen actives, vitamins, skin lightening agents, desquamation agents and herbal extracts.

9. Use as a moisturiser of a cosmetic composition according to any one of claims 1 to 5 or of a polymer having a plurality of acid groups at least partially neutralized by a mono-hydroxy substituted amine, the amine in non-protonated form having general formula I: 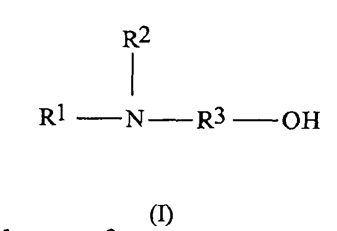
wherein R¹ and R² are branched or unbranched C₁-C₃₀ radicals selected from the group consisting of alkyl, cycloalkyl, alkenyl, aryl, alkylaryl, alkoxyalkyl and combinations thereof;
R³ is a branched or unbranched C₁-C₃₀ radical selected from the group consisting of alkylene, cycloalkylene, arylene and combinations thereof; and
R¹ with R² can optionally form a ring and independently R¹ with R³ can form a ring.

10. Use according to claim 9 wherein the amine is dimethylaminoethanol.

## Patentansprüche

1. Kosmetische Zusammensetzung, umfassend:
(i) etwa 0,000001 bis etwa 10 Gew.-% eines Polymers mit einer Vielzahl an Säuregruppen, die zumindest teilweise durch ein Monohydroxy-substituiertes Amin neutralisiert sind, wobei das Amin in nicht-protonierter Form die allgemeine Formel I aufweist: 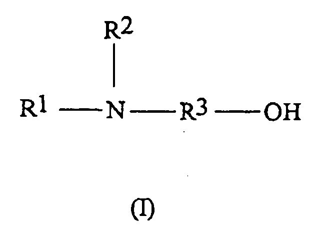
worin R¹ und R² verzweigte oder unverzweigte C₁-C₃₀-Reste sind, ausgewählt aus der Gruppe, bestehend aus Alkyl, Cycloalkyl, Alkenyl, Aryl, Alkylaryl, Alkoxyalkyl und Kombinationen davon;
R³ ein verzweigter oder unverzweigter C₁-C₃₀-Rest ist, ausgewählt aus der Gruppe, bestehend aus Alkylen, Cycloalkylen, Arylen und Kombinationen davon; und
R¹ mit R² gegebenenfalls einen Ring bilden kann und unabhängig R¹ mit R³ einen Ring bilden kann; und
(ii) 1 bis 99,9 Gew.-% eines kosmetisch verträglichen Trägers.

2. Zusammensetzung nach Anspruch 1, wobei das Amin Dimethylaminoethanol ist.

3. Zusammensetzung nach Anspruch 1 oder Anspruch 2, wobei der pH zwischen etwa 4 und weniger als etwa 7 liegt.

4. Zusammensetzung nach einem der vorhergehenden Ansprüche, wobei das Polymer aus einem Vinylmonomer gebildet ist, ausgewählt aus Acrylsäure, Methacrylsäure, Crotonsäure, Itaconsäure, Maleinsäure, Fumarsäure, 2-Acrylamido-2-methylpropan-sulfonsäure, 2-Methyl-2-[(1-oxo-2-propenyl)amino]-1-propansulfonsäure und Gemische davon.

5. Zusammensetzung nach Anspruch 4, wobei das Polymer außerdem aus einem Comonomer gebildet ist, ausgewählt aus Methylmethacrylat, Ethylacrylat, t-Butylacrylat, t-Butylmethacrylat, N-Vinylpyrrolidon, Acrylamid, Hydroxyethylacrylat, N-Hydroxypropylacrylamid, Vinylacetat, Vinylformamid, Styrol, Allylsaccharoseether, Divinylbenzol, Methylenbisacrylamid und Gemische davon.

6. Kosmetisches Haftpflaster oder ein Wegwerffeuchtigkeitstuch, enthaltend eine kosmetische Zusammensetzung nach einem der vorhergehenden Ansprüche

7. Zusammensetzung nach einem der Ansprüche 1 bis 5, wobei der Träger ein Silikonöl ist.

8. Zusammensetzung nach einem der Ansprüche 1 bis 5, außerdem umfassend eine Substanz, ausgewählt aus der Gruppe, bestehend aus Sonnenschutzwirkstoffen, Vitaminen, Hautaufhellungsmitteln, Schuppenmitteln und Kräuterextrakten.

9. Verwendung als ein Feuchthaltemittel einer kosmetischen Zusammensetzung nach einem der Ansprüche 1 bis 5 oder ein Polymer mit einer Vielzahl an Säuregruppen, die zumindest teilweise durch ein Monohydroxy-substituiertes Amin neutralisiert sind, wobei das Amin in nicht-protonierter Form die allgemeine Formel I aufweist: 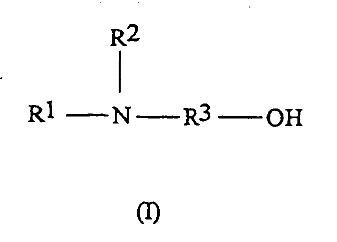
worin R¹ und R² verzweigte oder unverzweigte C₁-C₃₀-Reste sind, ausgewählt aus der Gruppe, bestehend aus Alkyl, Cycloalkyl, Alkenyl, Aryl, Alkylaryl, Alkoxyalkyl und Kombinationen davon;
R³ ein verzweigter oder unverzweigter C₁-C₃₀-Rest ist, ausgewählt aus der Gruppe, bestehend aus Alkylen, Cycloalkylen, Arylen und Kombinationen davon; und
R¹ mit R² gegebenenfalls einen Ring bilden kann und unabhängig R¹ mit R³ einen Ring bilden kann.

10. Verwendung nach Anspruch 9, wobei das Amin Dimethylaminoethanol ist.

## Revendications

1. Composition cosmétique comprenant
(i) de 0,000001 % environ à 10 % environ en poids d'un polymère présentant une pluralité de groupes acides au moins partiellement neutralisés par une lamine substituée par un mono-hydroxy, l'amine sous une forme non protonée ayant la formule générale I : 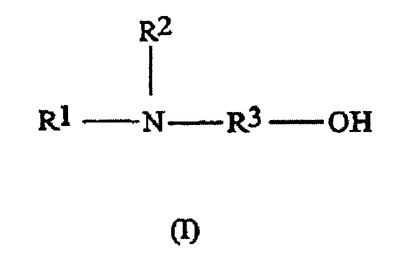
dans laquelle R¹ et R² sont des radicaux en C₁ à C₃₀ ramifiés ou non ramifiés choisis dans le groupe consistant en un groupe alkyle, un groupe cycloalkyle, un groupe alcényle, un groupe aryle, un groupe alkylaryle, un groupe alcoxyalkyle et des combinaisons de ceux-ci;
R³ est un radical en C₁ à C₃₀ ramifié ou non ramifié choisi dans le groupe consistant en un alkylène, en un cycloalkylène, en un arylène et en des combinaisons de ceux-ci et ;
R¹ avec R² peuvent optionnellement former un cycle et indépendamment R¹ avec R³ peuvent former un cycle ; et
(ii) de 1 à 99,9 % en poids d'un support cosmétiquement acceptable.

2. Composition selon la revendication 1 dans laquelle l'amine est le diméthylaminoéthanol.

3. Composition selon la revendication 1 ou la revendication 2 dans laquelle le pH s'inscrit dans une plage allant de 4 environ à moins de 7 environ.

4. Composition selon l'une quelconque des revendications précédentes dans laquelle le polymère est formé à partir d'un monomère de vinyle choisi parmi l'acide acrylique, l'acide méthacrylique, l'acide crotonique, l'acide itaconique, l'acide maléique, l'acide fumarique, l'acide 2-acrylamido-2-méthylpropane-sulfonique, l'acide 2-méthyl-2-[(1-oxo-2-propényl)amino]-1-propane sulfonique et des mélanges de ceux-ci.

5. Composition selon la revendication 4 dans laquelle le polymère est formé en outre d'un co-monomère choisi parmi le méthylméthacrylate, l'acrylate éthylique, l'acrylate t-butylique, le méthacrylate t-butylique, la N-vinylpyrrolidone, l'acrylamide, l'acrylate hydroxyéthylique, le N-hydroxypropylacrylamide, l'acétate vinylique, le formamide vinylique, le styrène; l'éther allylique de saccharose, le benzène de divinyle, le méthylène bisacrylamide et des mélanges de ceux-ci.

6. Tampon cosmétique adhésif ou produit de type lingette jetable contenant une composition cosmétique selon l'une quelconque des revendications précédentes.

7. Composition selon l'une quelconque des revendications 1 à 5 dans laquelle le support est une huile de silicone.

8. Composition selon l'une quelconque des revendications 1 à 5 comprenant en outre une substance choisie dans le groupe consistant en des substances actives en termes de protection solaire, des vitamines, des agents éclaircissants pour la peau, des agents de desquamation et des extraits d'herbe.

9. Utilisation en tant que crème hydratante, d'une composition cosmétique selon l'une quelconque des revendications 1 à 5 ou d'un polymère ayant une pluralité de groupes acides au moins partiellement neutralisés par une amine substituée par un mono-hydroxy, l'amine sous une forme non protonée ayant la formule générale I : 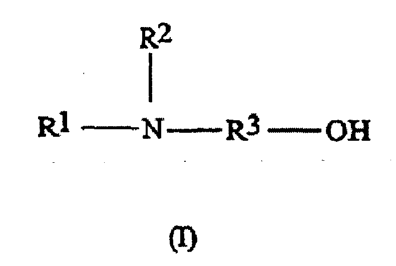
dans laquelle R¹ et R² sont des radicaux en C₁ à C₃₀ ramifiés ou non ramifiés choisis dans le groupe consistant en un groupe alkyle, un groupe cycloalkyle, un groupe alcényle, un groupe aryle, un groupe alkylaryle, un groupe alcoxyalkyle et des combinaisons de ceux-ci;
R³ est un radical en C₁ à C₃₀ ramifié ou non ramifié choisi dans le groupe consistant en un alkylène, en un cycloalkylène, en un arylène et en des combinaisons de ceux-ci et ;
R¹ avec R² peuvent optionnellement former un cycle et indépendamment R¹ avec R³ peuvent former un cycle.

10. Utilisation selon la revendication 9 dans laquelle l'aminé est le diméthylaminoéthanol.
